# FASCICULE DE BREVET EUROPEEN

(11) **EP 2 807 140 B1**
(45) Date de publication et mention de la délivrance du brevet: **28.10.2015**
(21) Numéro de dépôt: 13705205.6
(22) Date de dépôt: 14.01.2013
(51) Int. Cl.: C07C 67/03, C07C 67/54, C07C 69/54, C07C 67/293

(54) **PROCEDE DE PRODUCTION D'ACRYLATE DE 2-OCTYLE PAR TRANSESTERIFICATION**
VERFAHREN ZUR HERSTELLUNG VON 2-OCTYL-ACRYLAT DURCH UMESTERUNG
METHOD FOR THE PRODUCTION OF 2-OCTYL ACRYLATE BY MEANS OF TRANSESTERIFICATION

(30) Priorité: 23.01.2012 FR 1250607; 05.07.2012 FR 1256471
(43) Date de publication de la demande: 03.12.2014
(73) Titulaire: Arkema France, 92700 Colombes (FR)
(72) Inventeur: RIONDEL, Alain, F-77178 Saint Pathus (FR); GRAIRE, Coralie, F-69290 Grezieu-la-Varenne (FR); ESCH, Marc, F-57450 Theding (FR); LINEMANN, Reinhard, F-57200 Sarreguemines (FR)
(74) Mandataire: Bonnel, Claudine
(86) Numéro de dépôt international: PCT/FR2013/050078
(87) Numéro de publication internationale: WO 2013/110876

(56) Documents cités:
- US-B2- 6 977 310
- US-B2- 7 268 251
- DANNI LIU ET AL: "Rational Design of Pseudozyma antarctica Lipase B Yielding a General Esterification Catalyst", CHEMBIOCHEM, vol. 11, no. 6, 12 avril 2010 (2010-04-12) , pages 789-795, XP055033062, ISSN: 1439-4227, DOI: 10.1002/cbic.200900776

## Description

### DOMAINE DE L'INVENTION

La présente invention concerne la production d'acrylate de 2-octyle selon un procédé en continu par transestérification.

### ARRIERE-PLAN TECHNIQUE

Il est connu de produire des esters acryliques en mettant en oeuvre une réaction de transestérification entre un acrylate d'alcool léger (dénommé acrylate léger) et un alcool lourd.

Cette réaction est une réaction catalysée équilibrée avec génération d'alcool léger, selon la formule (I) :

CH₂=CH-COOR₁ + R₂-OH ⇔ CH₂=CH-COOR₂ + R₁-OH

Il est nécessaire d'éliminer l'alcool léger produit au cours de la réaction pour déplacer l'équilibre dans le sens de la production de l'ester acrylique.

Cette réaction s'accompagne généralement de réactions secondaires produisant des impuretés qu'il est nécessaire d'éliminer en vue d'obtenir l'ester acrylique avec une pureté élevée satisfaisant aux exigences techniques liées à son utilisation finale en tant que monomère pour fabriquer des polymères utilisables dans de nombreux domaines d'application.

Par ailleurs, pour des raisons économiques évidentes, les produits valorisables présents dans le mélange brut réactionnel, notamment les réactifs non réagis et le catalyseur, sont dans la mesure du possible recyclés au sein du procédé.

A ces fins, on procède généralement à un processus de séparation/purification comportant un ensemble de distillations, d'extractions, et/ou décantations, qui est à la fois relativement complexe à mettre en oeuvre, notamment du fait de la présence de mélanges azéotropiques, et coûteux sur le plan énergétique.

Différents procédés de transestérification pour produire des esters acryliques ont déjà été décrits dans l'art antérieur.

On peut citer par exemple le document US 7,268,251 dans lequel l'effluent réactionnel de la transestérification est traité de la façon suivante ;
soit on sépare tout d'abord la majeure partie de l'ester acrylique recherché et on l'isole ensuite du catalyseur utilisé par distillation (séparation de catalyseur),
soit on l'isole tout d'abord du catalyseur utilisé par distillation (séparation de catalyseur) et ensuite on sépare la majeure partie de l'ester acrylique,
et ensuite on sépare par distillation du mélange obtenu, les composés à point d'ébullition inférieur à celui de l'ester acrylique recherché (séparation des substances à bas point d'ébullition) et ensuite on distille l'ester acrylique (distillation à l'état pur).

Ce procédé nécessite la mise en oeuvre d'au moins quatre colonnes de distillation ou de rectification, dont un évaporateur pour séparer le catalyseur, généralement un alcoolate de titane.

Même si le procédé décrit dans le document US 7,268,251 concerne la fabrication d'acrylates d'alkyle par transestérification à partir d'un acrylate d'alkyle et d'un alcool présentant une longueur de chaine supérieure d'au moins un carbone par rapport à la chaine alkyle de l'acrylate de départ, ce procédé n'est illustré qu'avec la fabrication d'acrylate de diméthylaminoéthyle à partir de diméthylaminoéthanol et d'acrylate de méthyle ou d'acrylate d'éthyle dans une cascade de deux réacteurs.

Il s'avère que le procédé décrit dans le document US 7,268,251 est compliqué à mettre en oeuvre à l'échelle industrielle, du fait de l'optimisation des conditions opératoires de la succession des quatre éléments de distillation/rectification, pour obtenir un produit d'une grande pureté et une productivité satisfaisante.

Le document US 6,977,310 décrit un procédé de fabrication en continu d'esters alkyliques d'acide (méth)acrylique à partir de (méth)acrylate de méthyle et d'un alcool en C₂-C₁₂, en présence d'un titanate de tétraalkyle comme catalyseur de transestérification. Ce procédé consiste à soumettre le mélange réactionnel à une distillation sous pression réduite séparant les composés aisément volatils (réactifs non réagis) ; puis, la fraction résultante sortant en pied de colonne, comprenant l'ester produit, le catalyseur, les inhibiteurs de polymérisation et les produits secondaires à point d'ébullition élevé, est envoyée vers un étage de distillation sous vide qui permet de récupérer en tête l'ester produit de grande pureté. Selon ce procédé, illustré uniquement avec la fabrication de méthacrylate de butyle ou de méthacrylate d'isobutyle, le méthacrylate recherché se trouve dans le flux de pied de la première colonne de distillation sous pression réduite avant d'être séparé du catalyseur et purifié.

Le document EP 960 877, au nom de la Société Déposante, illustre un autre procédé de fabrication d'acrylate de diméthylaminoéthyle, plus généralement d'acrylate de dialkylaminoalkyle, par transestérification à partir de diméthylaminoéthanol et d'acrylate de méthyle ou d'acrylate d'éthyle.

Ce procédé consiste en une élimination du catalyseur et des produits lourds (équeutage), suivie d'une élimination des composés légers (étêtage) et d'une rectification finale d'un mélange brut réactionnel de transestérification obtenu à l'aide d'un catalyseur choisi parmi les titanates de tétrabutyle, de tétraéthyle et de tétra de (2-éthylhexyle). Ce procédé présente ainsi l'avantage de ne comporter que trois colonnes de distillation dans le train de purification du mélange réactionnel.

Cependant, le procédé décrit dans le document EP 960 877 n'est pas applicable à la fabrication d'acrylate d'alkyle à chaine longue, par exemple l'acrylate de 2-octyle par réaction de transestérification d'un acrylate léger avec du 2-octanol. En effet, la transestérification des titanates, soit avec l'alcool léger libéré au cours de la réaction, soit avec le 2-octanol de départ, provoque l'apparition d'impuretés telles que acrylate de butyle ou acrylate de 2-éthylhexyle, dans le mélange réactionnel ou dans le mélange azéotropique ester léger /alcool léger et complique la purification de l'acrylate de 2-octyle.

Il subsiste donc encore un besoin de disposer d'un procédé de fabrication d'acrylate de 2-octyle présentant une productivité compatible avec une fabrication à l'échelle industrielle et conduisant à un acrylate de 2-octyle répondant aux exigences de pureté liées à son utilisation finale, notamment quant à la possibilité d'utiliser ce monomère pour la fabrication de latex à faible teneur en composés organiques volatiles.

La Société Déposante a cherché à résoudre les différents problèmes des procédés précités, en particulier ceux liés à la mise en oeuvre du 2-octanol dans la réaction de transestérification, pour produire de l'acrylate de 2-octyle de très haute pureté avec un rendement élevé, tout en incluant le recyclage des produits valorisables tels que les réactifs non réagis et le catalyseur.

La solution proposée consiste à utiliser du titanate d'éthyle en solution dans le 2-octanol ou du titanate de 2-octyle comme catalyseur de transestérification, et à mettre en oeuvre un train de purification comportant une séparation préalable du catalyseur par distillation suivie d'une purification à l'aide d'au moins une colonne de distillation.

La présente invention permet en outre de produire un ester acrylique comportant du carbone d'origine renouvelable lié à l'utilisation du 2-octanol, qui est un alcool dérivé de matières végétales.

### RESUME DE L'INVENTION

La présente invention a pour objet un procédé de production en continu d'acrylate de 2-octyle par réaction de transestérification entre un acrylate d'alcool léger et du 2-octanol en présence d'un titanate d'alkyle comme catalyseur de transestérification et d'au moins un inhibiteur de polymérisation, le mélange azéotropique composé d'acrylate d'alcool léger et d'alcool léger généré par la réaction de transestérification étant soutiré en continu pendant la réaction, le mélange réactionnel étant soumis à un traitement de purification comportant au moins deux colonnes de distillation, afin d'obtenir d'une part l'acrylate de 2-octyle pur, d'autre part les composés 2-octanol et acrylate d'alcool léger non réagis destinés à être recyclés, et aussi le catalyseur destiné à être recyclé, procédé caractérisé en ce que :
- (i) on choisit le catalyseur parmi le titanate d'éthyle en solution dans le 2-octanol et le titanate de 2-octyle ;
- (ii) on effectue une séparation préalable du catalyseur en adressant à une première colonne de distillation (C1) sous pression réduite, le mélange brut réactionnel comprenant l'acrylate de 2-octyle recherché avec, comme produits légers, le 2-octanol et l'acrylate d'alcool léger n'ayant pas réagi, et comme produits lourds, le catalyseur, le ou les inhibiteurs de polymérisation ainsi que des produits de réaction lourds, et on effectue, dans ladite première colonne (C1), une distillation permettant d'obtenir ;
   o en tête, un flux composé essentiellement de l'acrylate de 2-octyle et des produits légers, comportant une fraction minoritaire d'inhibiteurs de polymérisation, mais exempt ou sensiblement exempt de catalyseur, et
   o en pied, un flux de produits de réaction lourds avec le catalyseur, le(s) inhibiteur(s) de polymérisation et une fraction minoritaire d'acrylate de 2-octyle, ledit flux étant recyclé à l'étape de réaction ; puis
- (iii) on sépare par distillation, du flux de tête de la première colonne de distillation (C1), les composés 2-octanol et acrylate d'alcool léger non réagis, une fraction comportant des inhibiteurs de polymérisation, et l'acrylate de 2-octyle pur ;
- (iv) on récupère l'acrylate de 2-octyle pur recherché;
- (v) on recycle les composés 2-octanol et acrylate d'alcool léger non réagis à l'étape de réaction ;
- (vi) on renvoie une fraction comportant des inhibiteurs de polymérisation à la colonne (C1) de séparation du catalyseur.

Selon un premier mode de réalisation de l'invention, l'étape (iii) du procédé est réalisée à partir des deux sous-étapes (iii1) et (iii2) suivantes :
- (iii1) on adresse le flux de tête de la première colonne de distillation (C1) à une seconde colonne de distillation (C2) sous pression réduite, dans laquelle est effectuée une distillation permettant d'obtenir :
   o en tête, un flux composé essentiellement de 2-octanol et d'acrylate d'alcool léger n'ayant pas réagi, avec une fraction minoritaire d'acrylate de 2-octyle ; et
   o en pied, l'acrylate de 2-octyle contenant des traces de produits non réagis et d'inhibiteurs de polymérisation ; puis
- (iii2) on adresse le flux de pied de la seconde colonne de distillation (C2) à une troisième colonne de distillation (C3) sous pression réduite, dans laquelle est effectuée une rectification permettant de séparer :
   ∘ en tête, l'acrylate de 2-octyle pur recherché ; et
   o en pied, les inhibiteurs de polymérisation en solution dans de l'acrylate de 2-octyle.

Selon un second mode de réalisation de l'invention, les étapes (iii) et (iv) du procédé sont réalisées simultanément en adressant le flux de tête de la première colonne (C1) à une seconde colonne (C2') sous pression réduite, dans laquelle est effectuée une distillation permettant d'obtenir :
∘ en tête, un flux composé essentiellement de 2-octanol et d'acrylate d'alcool léger n'ayant pas réagi, avec une fraction minoritaire d'acrylate de 2-octyle ;
o en pied, un flux contenant les inhibiteurs de polymérisation avec une fraction d"acrylate de 2-octyle ;
et de récupérer l'acrylate de 2-octyle pur recherché via un soutirage latéral.

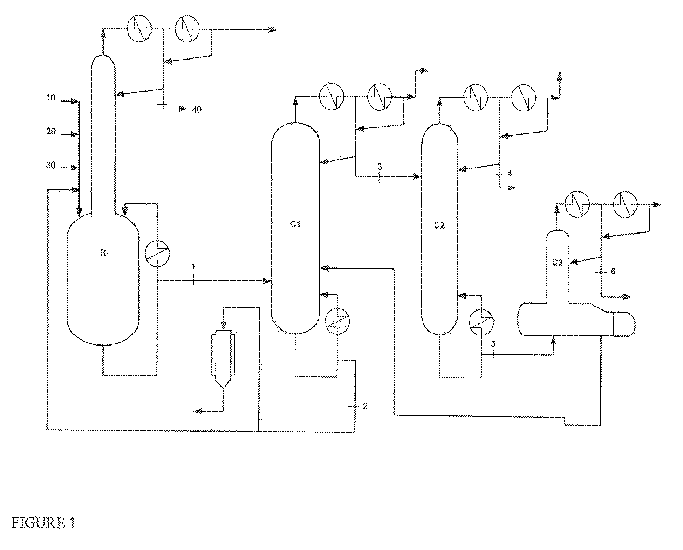

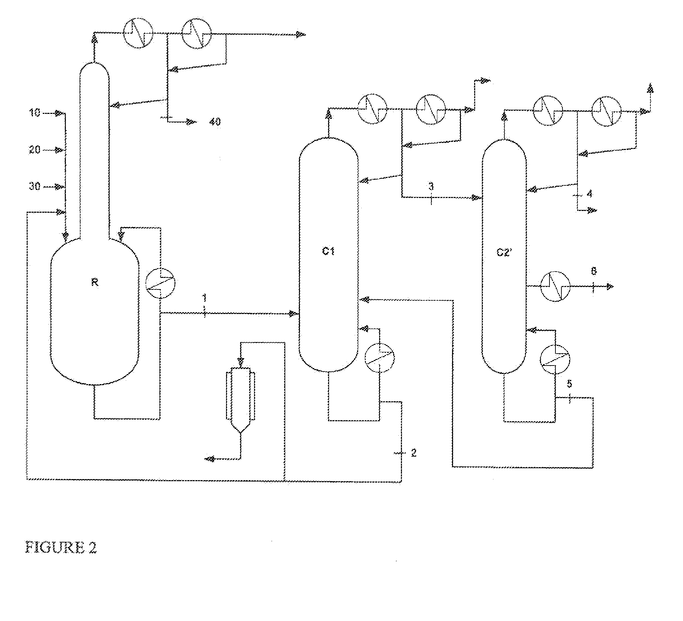

L'invention est maintenant décrite plus en détail et de façon non limitative dans la description qui suit, en référence aux figures 1 et 2 annexées qui représentent de manière schématique une installation permettant de mettre en oeuvre le procédé selon l'invention respectivement selon le premier mode et selon le second mode de réalisation décrits ci-dessus.

### DESCRIPTION DETAILLEE

L'un des objectifs de l'invention est d'utiliser des matières premières d'origine naturelle et renouvelable, c'est-à-dire biossourcées.

Le 2-octanol utilisé dans le procédé selon l'invention est un alcool d'origine renouvelable, en particulier il peut être obtenu par traitement alcalin de l'acide ricinoléïque dérivé de l'huile de ricin.

L'acrylate d'alcool léger mis en oeuvre comme matière première dans le procédé selon l'invention est obtenu par estérification directe de l'acide acrylique essentiellement produit sur le plan industriel à partir de propylène, avec un alcool léger, généralement le méthanol ou l'éthanol.

Indépendamment de la mise en oeuvre du 2-octanol d'origine renouvelable, l'invention s'étend à l'utilisation d'un acrylate d'alcool léger dérivé d'acide acrylique d'origine renouvelable, pouvant être en particulier obtenu à partir de glycérol, selon un procédé comportant une première étape de déshydratation du glycérol en acroléine suivie d'une étape d'oxydation en phase gaz de l'acroléine ainsi obtenue ; ou obtenu par déshydratation des acides 2-hydroxypropionique (acide lactique) ou 3-hydroxypropionique et de leurs esters.

L'invention s'étend également à l'utilisation d'un acrylate d'alcool léger dérivé d'un alcool biossourcé, tel que le bioéthanol.

D'une manière générale, la réaction de transestérification est réalisée dans un réacteur agité (R) avec un rapport molaire acrylate d'alcool léger / 2-octanol pouvant aller de 1 à 3, de préférence compris entre 1,3 et 1,8.

Comme acrylate d'alcool léger, on utilise l'acrylate de méthyle, l'acrylate d'éthyle ou l'acrylate de butyle, de préférence l'acrylate d'éthyle.

Le catalyseur de transestérification est le titanate d'éthyle en solution dans le 2-octanol, par exemple une solution à 90% de titanate d'éthyle dans le 2-octanol, ou le titanate de 2-octyle, obtenu préalablement par réaction à 100°C du titanate d'éthyle avec le 2-octanol), de préférence le titanate de 2-octyle.

On utilise le catalyseur à raison de 5.10⁻⁴ à 5.10⁻² mole par mole de 2-octanol, de préférence à raison de 10⁻³ à 10⁻² mole par mole de 2-octanol.

On conduit généralement la réaction de transestérification dans le réacteur (R) à une pression comprise entre 500 mm Hg (0,67.10⁵ Pa) et la pression atmosphérique, et à une température allant de 90°C à 130°C, de préférence de 100°C à 120°C.

La réaction est effectuée en présence d'un ou plusieurs inhibiteurs de polymérisation qui sont introduits dans le réacteur, à raison de 1000 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), le TEMPO (2,2,6,6-tétraméthyl-1-piperidinyloxy), le di-tertiobutylcatéchol, ou les dérivés du TEMPO, tel que le 4-hydroxy (OH)-TEMPO, seuls ou leurs mélanges en toutes proportions. Un ajout supplémentaire d'inhibiteur de polymérisation est généralement effectué au niveau du traitement ultérieur de purification, en particulier au niveau de chacune des colonnes de distillation.

L'alcool léger formé par la réaction de transestérification est entraîné en continu par distillation dans une colonne surmontant le réacteur sous forme d'un mélange azéotropique avec l'acrylate d'alcool léger.

Après réaction avec un temps de séjour dans le réacteur généralement compris entre 3 et 6 heures, le mélange brut réactionnel (1) contient l'acrylate de 2-octyle recherché avec, comme produits légers, le 2-octanol et l'acrylate d'alcool léger n'ayant pas réagi, et comme produits lourds, le catalyseur, le ou les inhibiteurs de polymérisation ainsi que des sous-produits de réaction lourds.

En référence à la figure 1, le mélange réactionnel est soumis à un traitement de purification pouvant comporter trois colonnes de distillation (C1), (C2) et (C3), afin d'obtenir d'une part l'acrylate de 2-octyle pur (colonne C3), d'autre part les composés 2-octanol et acrylate d'alcool léger non réagis destinés à être recyclés (colonne C2), et aussi le catalyseur destiné à être recyclé (colonne C1).

En variante et comme illustré par la figure 2, le traitement de purification ne comporte que deux colonnes de distillation (C1) et (C2') qui séparent le catalyseur destiné à être recyclé (colonne C1), l'acrylate de 2-octyle pur recherché et les composés 2-octanol et acrylate d'alcool léger non réagis destinés à être recyclés (colonne C2').

La première colonne de distillation (C1) fonctionne généralement sous une pression allant de 20 à 50 mm Hg (0,027 10⁵ Pa à 0,067 10⁵ Pa) à une température de pied allant de 120°C à 150°C.

Le flux (2) de pied de colonne (C1) est constitué du catalyseur, des sous-produits lourds, d'acrylate de 2-octyle et des inhibiteurs de polymérisation. Ce flux est avantageusement recyclé en partie à la réaction, l'autre partie étant éliminée via un évaporateur à film afin d'éviter une accumulation de fraction lourde dans l'installation.

Le flux (3) de tête de colonne (C1) est ainsi exempt ou sensiblement exempt de catalyseur et de produits lourds, et il est composé essentiellement de l'acrylate de 2-octyie recherché et des produits légers non réagis (acrylate d'alcool léger et 2-octanol),

Selon le premier mode de réalisation de l'invention illustré à la figure 1, ce flux (3) est soumis à une distillation dans une seconde colonne de distillation (C2) qui fonctionne généralement sous une pression de 20 à 50 mm Hg (0,027 10⁵ Pa à 0,067 10⁵ Pa) et une température de pied allant de 120°C à 150°C.

Le flux (4) de tête de la colonne (C2) est composé essentiellement de 2-octanol et d'acrylate d'alcool léger n'ayant pas réagi, avec une fraction minoritaire d'acrylate de 2-octyle ; il est avantageusement recyclé à la réaction.

En pied de la colonne (C2), on obtient dans un flux (5) l'acrylate de 2-octyle contenant des traces de produits non réagis et de sous-produits lourds, et le ou les inhibiteurs de polymérisation.

Le flux (5) est purifié sur une troisième colonne de distillation (C3) qui fonctionne généralement sous une pression allant de 20 à 50 mm Hg (0,027 10⁵ Pa à 0,067 10⁵ Pa) à une température allant de 120 à 150°C.

Les inhibiteurs de polymérisation séparés dans le flux de pied de la colonne (C3) sont avantageusement recyclés notamment au niveau de la colonne (C1).

L'acrylate de 2-octyle pur (6) est récupéré en tête de la colonne (C3). La pureté est supérieure à 99,5%, voire supérieure à 99,8%.

Selon le second mode de réalisation de l'invention illustré à la figure 2, le flux (3) est soumis à une distillation dans une seconde colonne de distillation (C2') qui fonctionne généralement sous une pression de 20 à 50 mm Hg (0,027 10⁵ Pa à 0,067 10⁵ Pa) et une température de pied allant de 120°C à 150°). La colonne (C2') est en général une colonne de distillation comportant de 15 à 25 plateaux théoriques.

Le fonctionnement de la colonne (C2') est modifié par rapport au fonctionnement de la colonne (C2) : ladite colonne (C2') est pourvue d'un soutirage latéral permettant d'extraire par la ligne 6, localisée à un niveau intermédiaire, le flux d'acrylate de 2-octyle pur recherché, et en tête le flux (4) composé essentiellement de 2-octanol et d'acrylate d'alcool léger n'ayant pas réagi, ce flux (4) étant avantageusement recyclé à la réaction.

Le flux (5), séparé en pied de la colonne (C2'), comporte essentiellement de l'acrylate de 2-octyle avec les inhibiteurs de polymérisation ; ce flux est avantageusement recyclé en pied de la colonne (C1).

Le soutirage latéral du produit pur recherché s'effectue en général en phase liquide ou en phase gazeuse, de préférence en phase gazeuse, à un niveau intermédiaire situé dans la partie inférieure de la colonne, en particulier entre les plateaux théoriques 14 et 24 pour un nombre de plateaux théoriques de 15 à 25 de la colonne.

Ce mode de réalisation est particulièrement avantageux, puisqu'il ne met en oeuvre qu'une seule colonne de distillation après la séparation du catalyseur et qu'il conduit à l'obtention d'un acrylate de 2-octyle de pureté supérieure à 99,8%.

Le recyclage du catalyseur, des produits réactifs non réagis ainsi que des inhibiteurs de polymérisation rend le procédé de l'invention particulièrement performant en termes de productivité.

Les exemples suivants illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AE : acrylate d'éthyle
A2OCT : acrylate de 2-octyle
PTZ : phénothiazine
EMHQ : ester méthylique d'hydroquinone

### Exemple 1 (selon l'invention)

Dans un réacteur R parfaitement agité, chauffé par un échangeur externe et surmonté d'une colonne à distiller de 12 plateaux théoriques, on charge de l'acrylate d'éthyle, du 2-octanol, un mélange de titanate d'éthyle en solution dans 2-octanol (mélange à 90 % de titanate d'éthyle dans le 2-octanol) avec de l'inhibiteur phénothiazine, dans les proportions massiques 53,8/45,6/0,6.

On met le réacteur en chauffe, sous bullage d'air et dès que la température atteint 115°C sous 500 mm Hg (0,67 10⁵ Pa), on introduit en continu l'AE stabilisé avec 2000 ppm de PTZ (10), le 2-octanol (20) et le titanate d'éthyle en solution dans le 2-octanol (30), dans des proportions massiques 53,8/45.6/0,6.

En tête de colonne, on soutire en continu l'azéotrope AE/Ethanol (40) avec une composition massique 35/65.

Le brut réactionnel (1), obtenu par réaction en continu, contient l'A2OCT, l'AE non réagi, le 2-octanol non-réagi et un mélange comprenant le catalyseur avec les inhibiteurs de polymérisation et des dérivés lourds, dans des proportions massiques 73/20, 1/6, 3/0,6.

Le flux (1) est envoyé en continu vers une première colonne de distillation C1 de 12 plateaux théoriques opérant sous pression réduite et chauffée par un échangeur externe à une température de 140°C.

En tête de colonne C1, on introduit un mélange à 2500 ppm de PTZ dans l'AE.

La colonne C1 sépare, en tête un mélange (3) AE/2-Octanol/A2OCT de composition massique 21/9/70, et en pied un mélange (2) comprenant les produits lourds, les inhibiteurs de polymérisation et le catalyseur et une fraction de A2OCT.

Le mélange (2) est renvoyé en partie à la réaction.

Le mélange (3) est envoyé vers une seconde colonne de distillation C2.

La colonne C2 de12 plateaux théoriques est chauffée par un échangeur externe et fonctionne sous un vide de 20 mm Hg (0,027 10⁵ Pa) en tête de colonne.

En tête de colonne C2, on introduit un mélange à 2500 ppm de PTZ dans l'AE.

La colonne C2 sépare en tête un mélange (4) AE/2-Octanol/A2OCT de composition massique 60/25/15 et en pied un mélange (5) enrichi en A2OCT. Le mélange (5) a la composition suivante :
- A2OCT 99.7%
- AE traces
- 2-Octanol 200 ppm
- PTZ 0,25%
- Autres 300 ppm

Le mélange (4) est recyclé à la réaction dans le réacteur R.

Le mélange (5) est envoyé vers une troisième colonne de distillation C3. La colonne C3 de 12 plateaux théoriques est chauffée par un échangeur et fonctionne sous un vide de 50 mm Hg (0,067 10⁵ Pa) en tête de colonne.

En tête de colonne C3, on introduit un mélange à 2500 ppm d'EMHQ dans l'A2OCT. La colonne C3 sépare en tête l'A2OCT (6) de pureté 99.85%, les réactifs restants étant présents à l'état de traces (2-octanol : 300 ppm).

Les inhibiteurs de polymérisation séparés en pied de la colonne C3 sont avantageusement recyclés à la colonne C1.

La faible teneur en 2-octanol contenue dans l'acrytate de 2-octyle pur est compatible avec la fabrication de latex à faible teneur en composés organiques volatiles.

### Exemple 2 (comparatif)

On a réalisé la même synthèse qu'à l'exemple 1, mais en utilisant comme catalyseur le titanate de butyle en remplacement du titanate d'éthyle.

Dans ce cas, le flux (4) distillé en tête de la colonne C2, contient outre les réactifs non réagis avec une fraction minoritaire de A2OCT, 15% d'acrylate de butyle provenant de la réaction du catalyseur avec l'AE.

Ce flux (4), destiné à être recyclé à l'étape réactionnelle, a nécessité une purification préalable par distillation sur une colonne supplémentaire pour éliminer l'acrylate de butyle, afin de limiter l'accumulation au cours du temps d'acrylate de butyle dans l'installation, et le risque de pollution de l'A2OCT purifié.

### Exemple 3 (comparatif)

On a réalisé la même synthèse qu'à l'exemple 1, mais en utilisant comme catalyseur le titanate de 2-éthylhexyle en remplacement du titanate d'éthyle.

Dans ce cas, on a obtenu en tête de la colonne C3, de l'A2OCT de pureté 97,5% en raison de la présence de 2% d'acrylate de 2-éthylhexyle provenant du catalyseur dans le produit purifié.

L'acrylate de 2-octyle ainsi obtenu n'offre pas les mêmes performances dans les adhésifs sensibles à la pression qu'un A2OCT de pureté 99,8%.

### Exemple 4 (comparatif)

On a reproduit l'exemple 1, mais en envoyant le brut réactionnel (1) directement vers la colonne de distillation C2, puis en envoyant la fraction de pied de la colonne C2 contenant le catalyseur vers la colonne C3.

Dans ce cas, on a obtenu de l'A2OCT contenant 500 ppm d'AE et 1500 ppm de 2-octanol, le catalyseur étant éliminé en pied de la colonne C3.

Une telle qualité de l'A2OCT ne convient pas pour les applications revêtement à très basse teneur en composés organiques volatiles.

### Exemple 5 (selon l'invention)

On a réalisé la même synthèse qu'à l'exemple 1 mais on a envoyé le flux 3 vers une colonne C2' comportant 15 plateaux théoriques munie d'un soutirage latéral au plateau 14.

On a obtenu un A2Oct pur de pureté 99,85%, les réactifs restants étant présents à l'état de traces (2-octanol : 300 ppm).

## Revendications

1. Procédé de production en continu d'acrylate de 2-octyle par réaction de transestérification entre un acrylate d'alcool léger et du 2-octanol en présence d'un titanate d'alkyle comme catalyseur de transestérification et d'au moins un inhibiteur de polymérisation, le mélange azéotropique composé d'acrylate d'alcool léger et d'alcool léger généré par la réaction de transestérification étant soutiré en continu pendant la réaction, le mélange réactionnel étant soumis à un traitement de purification comportant au moins deux colonnes de distillation, afin d'obtenir d'une part l'acrylate de 2-octyle pur, d'autre part les composés 2-octanol et acrylate d'alcool léger non réagis destinés à être recyclés, et aussi le catalyseur destiné à être recyclé, procédé **caractérisé en ce que** :
- (i) on choisit le catalyseur parmi le titanate d'éthyle en solution dans le 2-octanol et le titanate de 2-octyle ;
- (ii) on effectue une séparation préalable du catalyseur en adressant à une première colonne de distillation (C1) sous pression réduite, le mélange brut réactionnel comprenant l'acrylate de 2-octyle recherché avec, comme produits légers, le 2-octanol et l'acrylate d'alcool léger n'ayant pas réagi, et comme produits lourds, le catalyseur, le ou les inhibiteurs de polymérisation ainsi que des produits de réaction lourds, et on effectue, dans ladite première colonne (C1), une distillation permettant d'obtenir :
o en tête, un flux composé essentiellement de l'acrylate de 2-octyle et des produits légers, comportant une fraction minoritaire d'inhibiteurs de polymérisation, mais exempt ou sensiblement exempt de catalyseur, et
o en pied, un flux de produits de réaction lourds avec le catalyseur, le(s) inhibiteur(s) de polymérisation et une fraction minoritaire d'acrylate de 2-octyle, ledit flux étant recyclé à l'étape de réaction ; puis
- (iii) on sépare, par distillation, du flux de tête de la première colonne de distillation (C1), les composés 2-octanol et acrylate d'alcool léger non réagis, une fraction comportant des inhibiteurs de polymérisation, et l'acrylate de 2-octyle pur ;
- (iv) on récupère l'acrylate de 2-octyle pur recherché ;
- (v) on recycle les composés 2-octanol et acrylate d'alcool léger non réagis à l'étape de réaction ;
- (vi) on renvoie une fraction comportant des inhibiteurs de polymérisation à la colonne (C1) de séparation du catalyseur.

2. Procédé selon la revendication 1 **caractérisé en ce que** l'étape (iii) du procédé est réalisée à partir des deux sous-étapes (iii1) et (iii2) suivantes :
- (iii1) on adresse le flux de tête de la première colonne de distillation (C1) à une seconde colonne de distillation (C2) sous pression réduite, dans laquelle est effectuée une distillation permettant d'obtenir :
o en tête, un flux composé essentiellement de 2-octanol et d'acrylate d'alcool léger n'ayant pas réagi, avec une fraction minoritaire d'acrylate de 2-octyle ; et
o en pied, l'acrylate de 2-octyle contenant des traces de produits non réagis et des inhibiteurs de polymérisation ; puis
- (iii2) on adresse le flux de pied de la seconde colonne de distillation (C2) à une troisième colonne de distillation (C3) sous pression réduite, dans laquelle est effectuée une rectification permettant de séparer :
o en tête, l'acrylate de 2-octyle pur recherché ; et
o en pied, les inhibiteurs de polymérisation en solution dans de l'acrylate de 2-octyle.

3. Procédé selon la revendication 1 **caractérisé en ce que** les étapes (iii) et (iv) du procédé sont réalisées simultanément en adressant le flux de tête de la première colonne (C1) à une seconde colonne (C2') sous pression réduite, dans laquelle est effectuée une distillation permettant d'obtenir :
o en tête, un flux composé essentiellement de 2-octanol et d'acrylate d'alcool léger n'ayant pas réagi, avec une fraction minoritaire d'acrylate de 2-octyle ;
o en pied, un flux contenant les inhibiteurs de polymérisation avec une
fraction d"acrylate de 2-octyle et de produits non réagis ;
et de récupérer l'acrylate de 2-octyle pur recherché via un soutirage latéral.

4. Procédé selon l'une quelconque des revendications précédentes **caractérisé en ce que** le catalyseur est le titanate de 2-octyle.

5. Procédé selon l'une quelconque des revendications précédentes **caractérisé en ce que** l'on utilise le catalyseur à raison de 5.10⁻⁴ à 5.10⁻² mole par mole de 2-octanol, de préférence à raison de 10⁻³ à 10⁻² mole par mole de 2-octanol.

6. Procédé selon l'une quelconque des revendications précédentes **caractérisé en ce que** la réaction est réalisée à partir d'acrylate d'éthyle.

7. Procédé selon l'une quelconque des revendications précédentes **caractérisé en ce que** le rapport molaire acrylate d'alcool léger / 2-octanol va de 1 à 3, de préférence compris entre 1,3 et 1,8.

8. Procédé selon l'une quelconque des revendications précédentes **caractérisé en ce que** la réaction de transestérification est effectuée à une pression comprise entre 500 mm Hg (0,67 10⁵ Pa) et la pression atmosphérique (10⁵ Pa), et à une température allant de 90°C à 130°C, de préférence de 100°C à 120°C.

9. Procédé selon l'une quelconque des revendications précédentes **caractérisé en ce que** l'acrylate d'alcool léger est l'acrylate d'éthyle d'origine renouvelable.

## Patentansprüche

1. Verfahren zur kontinuierlichen Herstellung von 2-Octylacrylat durch Umesterungsreaktion zwischen einem Acrylat eines leichten Alkohols und 2-Octanol in Gegenwart eines Alkyltitanats als Umesterungskatalysator und mindestens eines Polymerisationsinhibitors, wobei die azeotrope Mischung, die aus Acrylat eines leichten Alkohols und durch die Umesterungsreaktion gebildetem leichtem Alkohol besteht, während der Reaktion kontinuierlich abgezogen wird, wobei die Reaktionsmischung einer Reinigungsbehandlung mit mindestens zwei Destillationssäulen unterworfen wird, wobei man einerseits reines 2-Octylacrylat, andererseits die zur Rezyklierung bestimmten nicht umgesetzten Verbindungen 2-Octanol und Acrylat eines leichten Alkohols sowie den zur Rezyklierung bestimmten Katalysator erhält, wobei das Verfahren **dadurch gekennzeichnet ist, dass**:
- (i) der Katalysator aus Ethyltitanat in Lösung in 2-Octanol und 2-Octyltitanat ausgewählt wird;
- (ii) eine Vorabtrennung des Katalysators durchgeführt wird, indem die rohe Reaktionsmischung, die das gewünschte 2-Octylacrylat mit dem nicht umgesetzten 2-Octanol und Acrylat eines leichten Alkohols als leichten Produkten und dem Katalysator, dem Polymerisationsinhibitor bzw. den Polymerisationsinhibitoren sowie schweren Reaktionsprodukten als schweren Produkten umfasst, einer ersten Destillationssäule (C1) unter vermindertem Druck zugeführt wird und in der ersten Säule (C1) eine Destillation durchgeführt wird, die es ermöglicht, Folgendes zu erhalten:
∘ am Kopf einen Strom, der im Wesentlichen aus 2-Octylacrylat und leichten Produkten besteht und eine untergeordnete Fraktion von Polymerisationsinhibitoren umfasst, aber zumindest weitgehend frei von Katalysator ist, und
∘ am Sumpf einen Strom von schweren Reaktionsprodukten mit dem Katalysator, dem Polymerisationsinhibitor bzw. den Polymerisationsinhibitoren und einer untergeordneten Fraktion von 2-Octylacrylat, wobei der Strom zur Reaktionsstufe rezykliert wird; dann
- (iii) durch Destillation des Kopfstroms aus der ersten Destillationskolonne (C1) die nicht umgesetzten Verbindungen 2-Octanol und Acrylat eines leichten Alkohols, eine Polymerisationsinhibitoren umfassende Fraktion und das reine 2-Octylacrylat getrennt werden;
- (iv) das gewünschte reine 2-Octylacrylat gewonnen wird;
- (v) die nicht umgesetzten Verbindungen 2-Octanol und Acrylat eines leichten Alkohols zur Reaktionsstufe rezykliert werden;
- (vi) der Säule (C1) eine Polymerisationsinhibitoren umfassende Fraktion zur Abtrennung des Katalysators zugeführt wird.

2. Verfahren nach Anspruch 1, **dadurch gekennzeichnet, dass** der Schritt (iii) des Verfahrens ausgehend von den folgenden zwei Unterschritten (iii1) und (iii2) durchgeführt wird:
- (iii1) der Kopfstrom aus der ersten Destillationssäule (C1) wird einer zweiten Destillationssäule (C2) unter vermindertem Druck zugeführt, in der eine Destillation durchgeführt wird, die es ermöglicht, Folgendes zu erhalten:
∘ am Kopf einen Strom, der im Wesentlichen aus nicht umgesetztem 2-Octanol und Acrylat eines leichten Alkohols mit einer untergeordneten Fraktion von 2-Octylacrylat besteht; und
∘ am Sumpf Octylacrylat, das Spuren von nicht umgesetzten Produkten und Polymerisationsinhibitoren enthält; dann
- (iii2) der Sumpfstrom aus der zweiten Destillationssäule (C2) wird einer dritten Destillationssäule (C3) unter vermindertem Druck zugeführt, in der eine Rektifikation durchgeführt wird, die es ermöglicht, Folgendes zu trennen:
∘ am Kopf das gewünschte reine 2-Octylacrylat; und
∘ am Sumpf die Polymerisationsinhibitoren in Lösung in 2-Octylacrylat.

3. Verfahren nach Anspruch 1, **dadurch gekennzeichnet, dass** die Schritte (iii) und (iv) des Verfahrens gleichzeltig durchgeführt werden, indem der Kopfstrom aus der ersten Säule (C1) einer zweiten Säule (C2') unter vermindertem Druck zugeführt wird, in der eine Destillation durchgeführt wird, die es ermöglicht, Folgendes zu erhalten:
∘ am Kopf einen Strom, der im Wesentlichen aus nicht umgesetztem 2-Octanol und Acrylat eines leichten Alkohols mit einer untergeordneten Fraktion von 2-Octylacrylat besteht; und
∘ am Sumpf einen Strom, der die Polymerisationsinhibitoren mit einer Fraktion von 2-Octylacrylat und nicht umgesetzten Produkten enthält;
und das gewünschte reine 2-Octylacrylat über einen Seitenabzug gewinnt.

4. Verfahren nach einem der vorhergehenden Ansprüche, **dadurch gekennzeichnet, dass** es sich bei dem Katalysator um 2-Octyltitanat handelt.

5. Verfahren nach einem der vorhergehenden Ansprüche, **dadurch gekennzeichnet, dass** man den Katalysator in einer Menge von 5·10⁻⁴ bis 5·10⁻² mol pro Mol 2-Octanol und vorzugsweise in einer Menge von 10⁻³ bis 10⁻² mol pro Mol 2-Octanol verwendet.

6. Verfahren nach einem der vorhergehenden Ansprüche, **dadurch gekennzeichnet, dass** die Reaktion ausgehend von Ethylacrylat durchgeführt wird.

7. Verfahren nach einem der vorhergehenden Ansprüche, **dadurch gekennzeichnet, dass** das Molverhältnis von Acrylat eines leichten Alkohols zu 2-Octanol im Bereich von 1 bis 3 und vorzugsweise zwischen 1,3 und 1,8 liegt.

8. Verfahren nach einem der vorhergehenden Ansprüche, **dadurch gekennzeichnet, dass** die Umesterungreaktion bei einem Druck zwischen 500 mm Hg (0,67·10⁵ Pa) und Normaldruck (10⁵ Pa) und einer Temperatur im Bereich von 90°C bis 130°C und vorzugsweise von 100°C bis 120°C durchgeführt wird.

9. Verfahren nach einem der vorhergehenden Ansprüche, **dadurch gekennzeichnet, dass** es sich bei dem Acrylat eines leichten Alkohols um Ethylacrylat erneuerbaren Ursprungs handelt.

## Claims

1. A process for the continuous production of 2-octyl acrylate by a transesterification reaction between a light alcohol acrylate and 2-octanol in the presence of an alkyl titanate as transesterification catalyst and at least one polymerization inhibitor, the azeotropic mixture composed of light alcohol acrylate and of light alcohol generated by the transesterification reaction being withdrawn continuously during the reaction, the reaction mixture being subjected to a purification treatment comprising at least two distillation columns, in order to obtain, on the one hand, the pure 2-octyl acrylate and, on the other hand, the unreacted 2-octanol and light alcohol acrylate compounds intended to be recycled, and also the catalyst intended to be recycled, which process is **characterized in that**:
- (i) the catalyst is chosen from ethyl titanate in solution in 2-octanol and 2-octyl titanate;
- (ii) a preliminary separation of the catalyst is carried out by sending, to a first distillation column (C1) under reduced pressure, the crude reaction mixture comprising the desired 2-octyl acrylate with, as light products, the unreacted 2-octanol and light alcohol acrylate and, as heavy products, the catalyst, the polymerization inhibitor or inhibitors and also heavy reaction products, and a distillation is carried out, in said first column (C1), which makes it possible to obtain:
o at the top, a stream composed essentially of 2-octyl acrylate and light products, comprising a minor fraction of polymerization inhibitors but devoid or substantially devoid of catalyst, and
o at the bottom, a stream of heavy reaction products with the catalyst, the polymerization inhibitor(s) and a minor fraction of 2-octyl acrylate, said stream being recycled to the reaction stage; then
- (iii) by distillation of the top stream from the first distillation column (C1), the unreacted 2-octanol and light alcohol acrylate compounds, a fraction comprising polymerization inhibitors and the pure 2-octyl acrylate are separated;
- (iv) the desired pure 2-octyl acrylate is recovered;
- (v) the unreacted 2-octanol and light alcohol acrylate compounds are recycled to the reaction stage;
(vi) a fraction comprising polymerization inhibitors is sent to the column (C1) for separation of the catalyst.

2. The process as claimed in claim 1, **characterized in that** stage (iii) of the process is carried out from the following two substages (iii1) and (iii2):
- (iii1) the top stream from the first distillation column (C1) is sent to a second distillation column (C2) under reduced pressure, in which a distillation is carried out which makes is possible to obtain:
o at the top, a stream composed essentially of unreacted 2-octanol and light alcohol acrylate, with a minor fraction of 2-octyl acrylate; and
o at the bottom, 2-octyl acrylate comprising traces of unreacted products and polymerization inhibitors; then
- (iii2) the bottom stream from the second distillation column (C2) is sent to a third distillation column (C3) under reduced pressure, in which a rectification is carried out which makes it possible to separate:
o at the top, the desired pure 2-octyl acrylate; and
o at the bottom, the polymerization inhibitors in solution in 2-octyl acrylate.

3. The process as claimed in claim 1, **characterized in that** stages (iii) and (iv) of the process are carried out simultaneously by sending the top stream from the first column (C1) to a second column (C2') under reduced pressure, in which a distillation is carried out which makes it possible to obtain:
o at the top, a stream composed essentially of unreacted 2-octanol and light alcohol acrylate, with a minor fraction of 2-octyl acrylate;
o at the bottom, a stream comprising the polymerization inhibitors with a
fraction of 2-octyl acrylate and of unreacted products;
and to recover the desired pure 2-octyl acrylate via a side stream.

4. The process as claimed in any one of the preceding claims, **characterized in that** the catalyst is 2-octyl titanate.

5. The process as claimed in any one of the preceding claims, **characterized in that** the catalyst is used in a proportion of 5 × 10⁻⁴ to 5 × 10⁻² mol per mole of 2-octanol, preferably in a proportion of 10⁻³ to 10⁻² mol per mole of 2-octanol.

6. The process as claimed in any one of the preceding claims, **characterized in that** the reaction is carried out starting from ethyl acrylate.

7. The process as claimed in any one of the preceding claims, **characterized in that** the light alcohol acrylate/2-octanol molar ratio ranges from 1 to 3, preferably between 1.3 and 1.8.

8. The process as claimed in any one of the preceding claims, **characterized in that** the transesterification reaction is carried out at a pressure of between 500 mmHg (0.67 × 10⁵ Pa) and atmospheric pressure (10⁵ Pa) and at a temperature ranging from 90°C to 130°C, preferably from 100°C to 120°C.

9. The process as claimed in any one of the preceding claims, **characterized in that** the light alcohol acrylate is ethyl acrylate of renewable origin.
